**Europäisches Patentamt**

**European Patent Office**

**Office européen des brevets**

(11) Publication number: **0 125 067**

**A1**

# EUROPEAN PATENT APPLICATION

) Application number: **84302795.4**

) Date of filing: **25.04.84**

(51) Int. Cl.³: **C 10 L 1/00**
//C07C9/14, C07C31/04,
C07C43/04, C07C1/02, C07C2/80,
C07C29/15, C07C41/01

) Priority: **04.05.83 GB 8312172**

) Date of publication of application:
**14.11.84 Bulletin 84/46**

) Designated Contracting States:
**AT BE CH DE FR GB IT LI LU NL SE**

(71) Applicant: **ENERGY CONCEPTS LIMITED**
**Piermont House 33-35 Pier Road**
**St Helier Jersey Channel Islands(GB)**

(72) Inventor: **Willis, James David**
**13 Southern Way**
**Letchworth Hertfordshire SG6 4TD(GB)**

(74) Representative: **Oliver, Roy Edward et al,**
**POLLAK MERCER & TENCH High Holborn House 52-54**
**High Holborn**
**London WC1V 6RY(GB)**

) Liquid fuel production and energy conversion systems.

) A process and apparatus are disclosed for converting rganic material, especially organic waste matter, into a liquid omposition useful as a fuel, which is suitable for the operaon of diesel and other i.c. engines. The apparatus consists of feedstock charging unit 11 which introduces solid finely-ivided organic matter into a sealable first reactor 14, where asification occurs and an autogenous pressure is built up ntil a valve 16 is opened and the first gas mixture passes to a rst catalytic treatment zone 18 and from there to a second atalytic treatment zone 26, the gas product from which is ien subjected to a silent electric discharge in an electrostatic nergy field unit 29 prior to undergoing reduction to normal ressure and temperature in a condenser 32. The resultant roduct is a liquid composition. In a preferred embodiment, peration with an initial solid feedstock alternates with operaon with a gaseous feedstock (Figure 3b) which produces an dditional quantity of liquid product, prior to resumed operaon with a further solid feedstock charge.

FIG.2.

- 1 -

LIQUID FUEL PRODUCTION AND ENERGY CONVERSION SYSTEMS

DESCRIPTION

This invention relates to the production of a liquid composition which is useful as a fuel and is concerned in particular with methods and apparatus for the production of such liquid compositions and their use as fuels, for instance for operating internal combustion engines. The invention is based upon a series of discoveries in relation to the anaerobic thermal decomposition of organic matter, especially waste vegetable matter. Major advantages of the invention include both the disposal of virtually any waste organic matter and also the efficient production of an easily-utilizable fuel and consequently the efficient generation of energy. The process of the invention avoids the production of significant amounts of solid residues or other by-products which would give rise to disposal problems. Moreover, the liquid fuel produced in carrying out the invention and also various intermediate organic gaseous compositions can supply many times the energy required by the process itself, so that the invention can be realised as a total energy system having a substantial positive output, by utilizing a portion of the liquid fuel or a bleed-off or recycle of a proportion of the gases produced in carrying out the process to generate the thermal, electrical and kinetic energy required by the process.

The initial step involved in the process of the invention is the gasification of organic matter at elevated temperature while excluding air, so that the organic matter is first put into a physical form suitable for gasification and then subjected to anaerobic thermal decomposition. Many known processes in which vegetable waste is treated have an initial step of this kind, including the destructive distillation of wood, which was the main source of methanol or "wood alcohol" before synthetic methods were developed, based mainly on the catalytic hydrogenation of carbon monoxide under elevated temperature and pressure. As indicated in more detail below, the initial step in the process of the invention involves thermal decomposition of organic matter under conditions of elevated temperature and elevated pressure, with the exclusion of atmospheric oxygen.

According to an aspect of this invention, a process of production of a liquid composition, useful as a fuel, comprises:

(a) subjecting a first organic gas mixture to at least one stage of catalytic treatment at elevated temperature and elevated pressure so as to cause interaction of the components of the first gas mixture and formation of a second organic gas mixture, and

(b) exposing the second gas mixture to an electrostatic energy field of the silent discharge type, wherein steps (a) and (b) are each conducted in the substantial absence of molecular oxygen, step (b) is conducted at an elevated pressure not greater than the maximum pressure attained in step (a) and step (a) is conducted at an elevated temperature not less than that used in step (b). The first gas mixture is preferably produced by subjecting finely-divided solid organic matter to decomposition at elevated temperature under autogenous pressure so as to effect substantially complete gasification of the organic matter; the gasification step is conducted in the absence of molecular oxygen, like steps (a) and (b).

In accordance with an important and preferred embodiment of the process of the invention, step (a) comprises a first catalytic treatment of the first gas mixture in the presence of a catalyst comprising calcium oxide and alumina and a second catalytic treatment of the resultant product in the presence of a catalyst comprising calcium oxide, carbon and metallic copper, so as to form the second gas mixture, whereby the product of the first treatment has a higher range of number of carbon atoms in the molecule than the first gas mixture and the second gas mixture has a still higher range of number of carbon atoms.

In accordance with another important feature of the process of the invention, the process is carried out by a multiple batch procedure, in which a first charge of solid organic matter is subjected to fine sub-division, if necessary, and is then subjected to treatment in a reaction zone, until the gasification step has been completed and the first gas mixture so formed is then transferred to a second reaction zone in order to undergo step (a). Any solid residue from the first charge which remains in the reaction zone can either be removed, for instance after returning the reaction zone to a lower pressure, or it can remain, subject to the capacity of the vessel forming the reaction zone. In either case, a second charge of solid organic matter is then introduced into the reaction zone and the process is then repeated. In this way, charges of organic matter can be processed on what is essentially a continuous basis. The necessity to exclude oxygen from the apparatus in which the process is carried out and the importance of the build-up of the autogenous pressure in the gasification step mean that a multiple batch type of operation, as opposed to a wholly continuous procedure, has many practical advantages. The invention includes a further and highly advantageous method of operation, which avoids the need to remove a solid residue from the reaction zone each time gasification has been carried out, or at least, when this step has been carried out a number of times so that the residues occupy the space available. In accordance with this preferred embodiment of the invention, after gasification has been completed and while the reaction zone remains at the elevated temperature or is maintained at another elevated temperature and also while the reaction zone and the residue in it are subject to an elevated pressure derived from the autogenous pressure of the gasification step, a combustible organic gas feed is supplied to the reaction zone and reaction of it with the essentially carbonaceous residue yields a quantity of another gas mixture, which then undergoes steps (a) and (b), resulting in the production of additional liquid composition and substantial removal of the residue from the reaction zone. When operation using this gas feed is completed, a further batch of organic matter is then introduced into the reaction zone and is subjected to gasification. This especially advantageous embodiment of the process thus involves the carrying out of these three steps repeatedly and each gasification operation alternates with an operation using this gas feed. The gas feed step is essentially an alternative way, in comparison with the gasification of organic matter of a comminuted solid form, to produce a first gas mixture, prior to subjecting it to the one or more catalytic treatments and the electrostatic energy field of steps (a) and (b).

In carrying out the proecess of the invention, the organic matter constituting the feedstock can be of virtually any composition of animal or vegetable origin. Waste vegetable matter and vegetable products generally are very suitable for carrying out the invention, as they can usually be subjected to fine sub-division without difficulty, in preparation for the gasification step.  Also, waste materials would otherwise represent a disposal problem.  Coal and other essentially organic carboniferous materials are also suitable starting materials for the process of the invention.  It is necessary to be able to introduce the feedstock into a reaction zone, such as a furnace chamber or other heatable enclosure, in the absence of oxygen and the feedstock is therefore most conveniently a material in or capable of being processed to a flowable or pumpable state.  Organic vegetable matter such as wood, leaves, grasses, potatoes, root vegetables, straw or the like is preferably ground or chopped to a fine particle size range.  Metallic or mineral matter such as stones should be excluded, as well as any animal or vegetable materials which are too hard or coarse to be suitable for fine grinding.

The gasification step is preferably carried out in a reaction zone, for example, an electric furnace chamber, maintained at a temperature in the range from $1000^{\circ}$ to $1500^{\circ}C$ and, preferably from $1000^{\circ}$ to $1200^{\circ}C$. In one form of apparatus suitable for forming the reaction zone, the chamber is preferably lined with or fitted with electrical heating elements embedded in $CaO_2$ slabs or blocks.  The chamber has to be evacuated so as to exclude molecular oxygen.  In practice, the entire apparatus is evacuated so that the atmosphere in it during the steps of the process is essentially the mixture of gases produced in the gasification step or the gaseous or liquid products derived from it as the later steps are carried out.  The feedstock can be advantageously introduced into the pre-heated chamber, on starting up the process, at a pressure in the range of 2000 to 3000 psi or 125 to 200 atms (approximately 14,000 to 20,000 $kN/m^2$).  Introduction can be made through an injector of the kind used in a Diesel engine, but, in accordance with a preferred embodiment of the invention, the feedstock is introduced in the reaction zone by purpose-built apparatus which carries out the necessary treatment of the charge of organic material to put it into a form suitable for the gasification step. Introduction of the feedstock is continued and gasification commences, the gaseous and vapour products forming the first gas mixture being retained in the furnace chamber until the pressure reaches a predetermined value, for instance in the range from 500 to 1000 psi and preferably 700 psi (3500 to 7000 and preferably 5000 $kN/m^2$); this predetermined autogenous pressure should not be less than 450 psi or 30 atms (3000 $kN/m^2$) and when the selected pressure has been attained, a pressure-sensitive valve is opened and the gas/vapour mixture produced is discharged from the furnace. The introduction of feedstock, its gasification and discharge of the gas/vapour product at the selected predetermined pressure can proceed indefinitely, when operating according to a continuous or multiple batch form of the process, as described in more detail below. It has been found that the treatment of organic matter under these conditions leads to its virtually total gasification. The product of this step in the process is a gaseous or vapour-phase mixture which consists mainly of water vapour and carbon monoxide with small amounts of carbon dioxide and traces of ammonia; the actual composition will obviously depend upon the nature of the organic materials in the charge fed to the reaction zone in order to carry out the gasification step.

The gas/vapour mixture passes via the pressure-sensitive valve to a second reactor or, preferably, to the first of two successive reactors, in which or in each of which catalytic reaction of the components of the mixture occurs. In a preferred form of the process, the first gas mixture is fed into a reaction zone in a reactor maintained at a temperature in the range from $300^{\circ}$ to $750^{\circ}C$, preferably at $500^{\circ}C$, and at a pressure in the range from 450 to 900 psi or 30 to 60 atms (3000 to 6000 $kN/m^2$). In any case, the second reaction zone is operated so that the reaction step carried out in it is conducted at a pressure which is equal to or below the maximum autogenous pressure developed in the gasification step and also at a temperature which is equal to or below that used in the gasification step. As a general rule, the temperature and pressure used are below those used in the gasification step. The catalyst comprises calcium oxide, CaO, and alumina, $Al_2O_3$, preferably in the proportion of 50-70% and most preferably 55% of CaO and 30 - 50% and most preferably 40% of $Al_2O_3$, by weight, together with a small amount, say 1 - 5% by weight of powdered graphite. The catalyst is preferably made by admixing the selected components in a uniform manner and the mass can then be pressed to form a round billet or block, having a plurality of passageways bored or otherwise formed lengthwise in it. Alternatively and preferably, the catalyst mass is in granular or particulate form and the reaction chamber is arranged so as to effect maximum contact between the gas mixture and the catalyst. This also applies naturally to the second and any other catalytic treatment stage.

Use of solid multiperforate catalyst blocks is one of a number of ways in which a catalyst component of high surface area can be provided so as to allow the gas/vapour mixture to pass through and undergo the desired reaction. The effects of the temperature, pressure and catalyst on the gas/vapour mixture produce a vapourous product which appears typically to comprise methyl ether, $CH_3OCH_3$, and methanol, $CH_3OH$, derived from hydrogenation

and other reactions between the water, CO, $CO_2$, $N_2$ and other components and their products of decomposition. The resultant reaction product mixture is then treated, e.g. in a second and similar reactor, where the catalyst is of similar shape and construction, but comprises CaO, powdered graphite and finely-divided metallic copper. These catalyst components are preferably present in amounts ranging from 30-50% and preferably 40%, in the case of each of the calcium oxide and the copper, and from 15-30% and preferably 20% in the case of the graphite, by weight. The second reactor is preferably operated at a lower temperature than the first, e.g. $250^{O}-350^{O}C$ and preferably $300^{O}C$, while the pressure can be in the same range as in the first catalytic reactor chamber. The effect of the second thermal catalytic treatment appears to be to initiate some measure of reaction between the simple organic substances and the nitrogen, so that a much denser vapour product arises. Typically, the product of the first catalytic treatment step contains organic compounds having from 1 to 4 carbon atoms in the molecule and the effect of the second catalytic step is the cracking and reformation of these compounds into a range of substances of larger molecular size, typically comprising from 5 to 8 carbon atoms, in conjunction with other, non-organic components.

In the next stage in the process, the product is preferably first cooled, typically to $100^{O}C$ to $200^{O}C$ and preferably to $150^{O}C$, which causes the product to remain substantially in vapour form. The product is then passed into another reaction chamber fitted with elements for the production of an electrostatic energy field, e.g. at 25,000 to 40,000 volts DC and preferably at 30,000 volts DC. The energy field chamber is maintained under pressure at the temperature to which the product has been cooled and, further condensation and possibly nitration of the components of the product is apparently caused by this further treatment step. The organic compounds in the product obtained after the gas mixture has undergone the energy field step have a range of molecular sizes greater than those in the product subjected to this treatment. Typically, the organic compounds in the product are mainly in the range from $C_6$ to $C_{12}$ in terms of the numbers of carbon atoms present. Each stage after the gasification step appears to increase the molecular size of at least the organic components present and this is responsible for the ultimate formation of a complex product which is largely organic and which becomes a liquid when reduced to normal temperature and pressure.

The product is then cooled to ambient temperature or, in general, a temperature in the range from $10^{\circ}$ to $25^{\circ}C$, preferably $20^{\circ}C$ and typically comprises a liquid mixture, the main components of which are methyl ether, which may constitute 55% to 75% and usually about 60% by volume of the product, and methanol, which may constitute most of the remainder. The actual make-up of the product depends upon the material used, as will be readily understood. Conversion of the feedstock to a liquid mixture in this way can readily be achieved with a conversion efficiency of 95% or even higher. The product is eminently suitable for use as a fuel for internal combustion engines operating either by carburation, fuel injection or on the Diesel principle. Operation of the overall process can be conducted so as to use 15 kW of energy, for instance, and it has been found that by using the most preferred conditions at each stage enough fuel is made to generate 60 kW of energy. Thus, the product of the process can yield enough fuel to operate the entire system and also an excess amount of fuel capable of generating as much as 3 times the amount of energy as is required by the apparatus in which the process is conducted.

The process also consists in an apparatus for producing a liquid composition useful as a fuel from organic matter and comprises:

(a) a first reaction zone capable of being evacuated and heated, having a sealable inlet for the introduction of a charge of organic matter, heating of which causes gasification and the formation of a first gas mixture under the autogenous pressure of such gasification step, and a sealable outlet for effecting discharge of the first gas mixture from the first reaction zone,

(b) a second reaction zone capable of being heated and arranged to contain at least one catalyst substance for effecting reaction of the components of the first gas mixture to form a second gas mixture, the second reaction zone having an inlet for introduction of the first gas mixture connected to the sealable outlet from the first reaction zone and an outlet for effecting discharge of the second gas mixture from the second reaction zone,

(c) a third reaction zone including means for generating an electrostatic energy field of the silent discharge type and having an inlet for introduction of the second gas mixture connected to the outlet from the second reaction zone and an outlet for effecting discharge of a third gas mixture produced by the action of the energy field upon the second gas mixture as it passes through the third reaction zone,

(d) a condenser connected to the outlet from the third reaction zone and serving to effect reduction of the temperature of the third gas mixture to produce the desired liquid composition and

(e) means for connecting the first, second and third reaction zones and the condenser to a source of vacuum.

In order that the invention may be fully understood, a producer plant comprising a first embodiment of apparatus for carrying out the process, a flow-sheet of the main steps of the process and an apparatus comprising a second and preferred embodiment of apparatus according to the invention are described below, by way of illustration in the non-limitative accompanying drawings, in which:

Fig. 1 shows an apparatus according to a first embodiment of the invention, in diagrammatic view;

Fig. 2 shows in flow-sheet form the steps of the process of the invention;

Fig. 3 comprises Figs. 3a, 3b and 3c taken together and showing an apparatus according to a further and preferred embodiment of the apparatus of the invention, in sectional view, where

Fig. 3a shows a feedstock preparation and introduction unit, which also serves to seal the first reaction zone when a feedstock charge has been formed and introduced;

Fig. 3b shows a two-purpose unit comprising a thermal decomposition chamber containing the first reaction zone and serving to effect gasification under autogenous pressure of the feedstock charge supplied to it, the thermal decomposition chamber being located for heat economy purposes within a first catalytic treatment chamber containing a first catalyst and forming a second reaction zone;  and

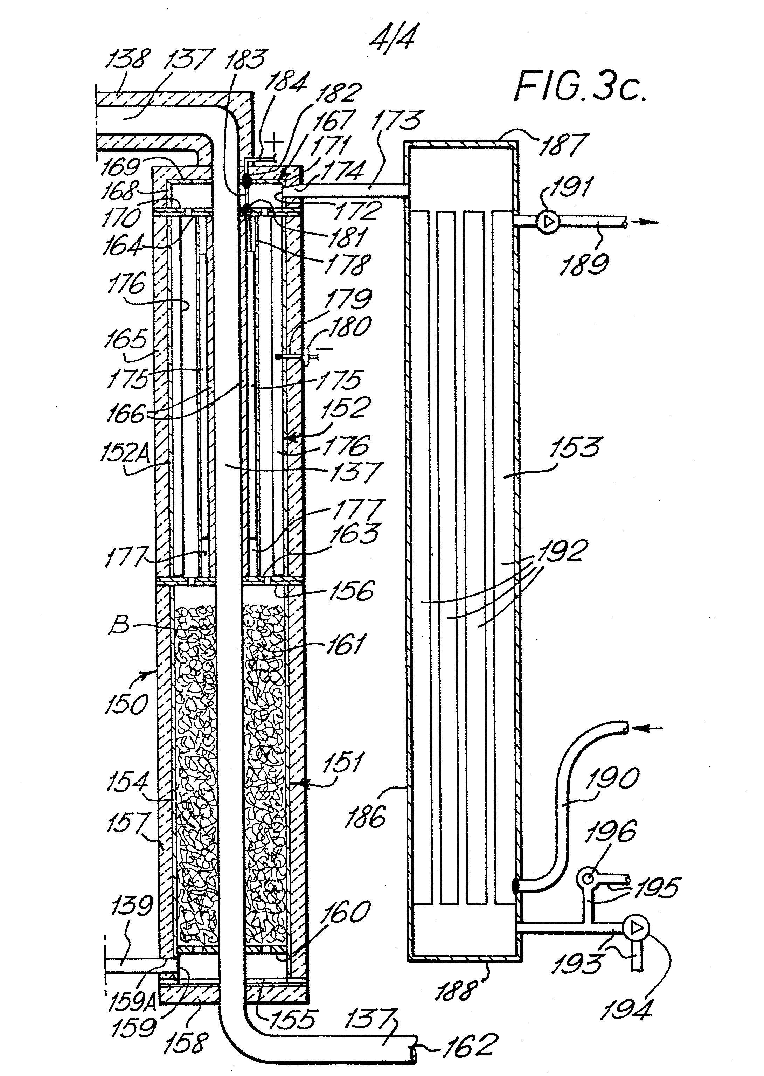

Fig. 3c shows a two-purpose unit comprising a first part including a second catalytic treatment chamber containing a second catalyst and forming a third reaction zone, arranged to receive the gas product from the first catalytic treatment and subject it to another catalytic treatment step and a second part including a fourth reaction zone equipped with electrodes for generating an electrostatic energy field, both parts of the unit being traversed by a heat exhaust pipe for maintenance of the desired temperature and also being connected in series to the outlet from the first catalytic treatment chamber, together with a coolant-supplied condenser connected to the outlet from the fourth reaction zone and itself having an outlet for directing the liquid composition to a product storage arrangement.

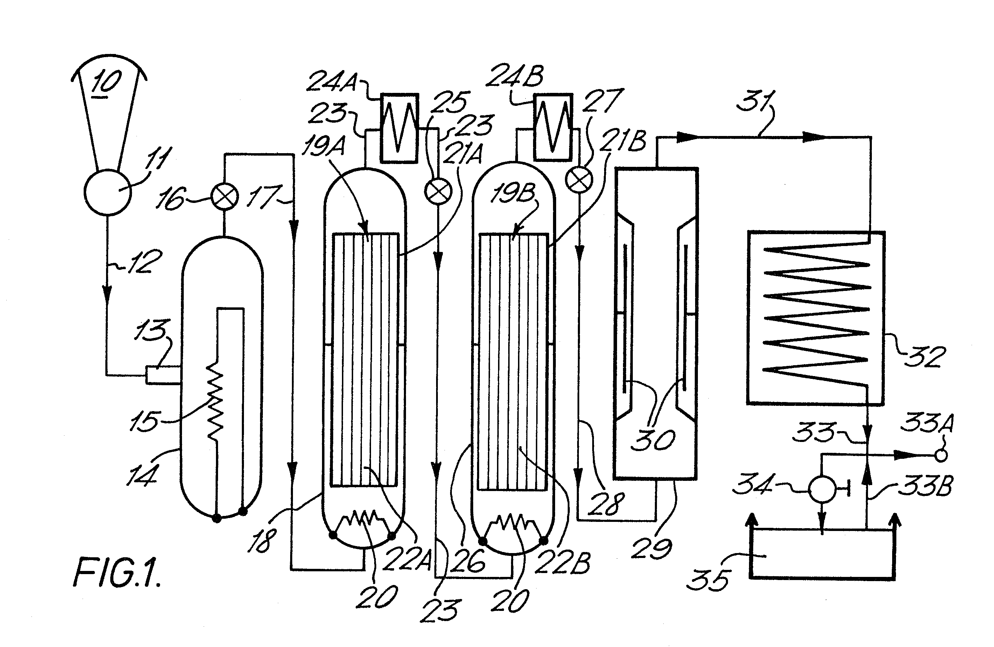

Referring to Fig. 1, organic feedstock in the form of raw vegetation, coal, wood or other primarily vegetable and/or animal matter is first prepared by collecting it in a feedstock storage arrangement 10 and grinding it to a fine pulp or other finely sub-divided form in a grinder or pulping unit 11. This feedstock is fed via line 12 to an injector 13, by means of which it is then injected into an evacuated chamber 14. The injection step can be effected at high pressure or, alternatively, at a lower pressure, e.g. atmospheric, so that the chamber 14 is then closed after a suitable quantity has been introduced. In the chamber 14, the charge is totally gasified at high temperature, as indicated above, the temperature being maintained by, for instance, electric heating elements, such as are indicated diagrammatically at 15. In the chamber 14, the gasification produces a gaseous product containing carbon monoxide and water vapour, together with small amounts of carbon dioxide, ammonia and free nitrogen. The injection of the feedstock via the injector 13 or otherwise continues until the gasification produces a pressure within the chamber 14 preferably in the range from 30-40 atms (3000-4000 $kN/m^2$). At a predetermined pressure typically above 30 atms, (3000 $kN/m^2$), e.g. 35 atms (350 $kN/m^2$), the gaseous products are then vented through a valve 16 into a line 17 leading to a further evacuated chamber 18 containing a catalyst 19A and heated by elements indicated diagrammatically at 20.

The catalyst 19A comprises a mixture of 55% calcium oxide and 40% alumina thoroughly mixed together, with 5% powdered graphite, to provide a uniform mix which is then pressed into a round billet or it is formed into a gas-permeable mass of particles. The catalyst 19A is heated to a temperature of $500^{\circ}C$. As the gases enter the chamber 18 and the pressure increases to that of the gasification chamber 14, i.e. 30-40 atmospheres, the gaseous products are forced through the catalyst 19A where a reaction occurs producing vapours containing $CH_3-O-CH_3$ plus $CH_3-OH$ and free nitrogen. This product is then passed through a line 23 and a cooling unit 24A via another pressure-sensitive valve 25 to a further catalyst chamber 26 constructed in the same way as the chamber 18. The catalyst 19B is similar to the catalyst 19A, but comprises 40% of calcium oxide, 20% of powdered graphite and 40% of finely-divided copper. The catalyst 19B is also heated to $350^{\circ}C$ and the chamber 26 is also maintained under a pressure of 30 atmospheres.

The reactions occurring at this stage link the free nitrogen molecules to the $CH_3-O-CH_3$ or $CH_3-OH$ molecules, producing a much denser vapour, which is apparently slightly nitrated. This vapour is then sub-jected to cooling to $150^{\circ}C$ in a second cooling unit 24B and it is then passed via a valve 27 and a line 28 to a chamber 29 in which elements 30 establish a high-intensity electrostatic field of a silent discharge nature. From the chamber 29, the product is passed via a line 31 to a condenser 32 in which it is cooled to around $20^{\circ}C$, producing a solution comprising $CH_3-O-CH_3 + CH_3-OH$. This is then fed via line 33 to an outlet 33A, leading to a storage tank for instance, or alternatively the line 33 passes the solution via a control valve 34 to a nitration vessel 35, where the product is further nitrated, to produce a final product of nitrated $CH_3-O-CH_3 + CH_3-OH$, which is then returned via line 33B to the outlet 33A.

The whole process is dependent upon a total pressure throughout of not less than 30 atmospheres, which is produced initially by the gasification step in the chamber 14. Air must be excluded at all times, in order to exclude molecular oxygen and therefore to prevent contamination of the final product and oxidation of the catalysts 19A and 19B. It is also essential for the catalyst compositions to be correctly maintained, as alteration of the constituents and/or their percentages can affect the final product.

The precise nature of the reactions which take place as the feedstock undergoes the successive steps of the form of the process which takes place in the apparatus illustrated in Fig. 1 has not been determined fully. However, it is believed that the general reaction sequence is as described below, where the typical values of the main operating conditions are also set out. The temperatures indicated for the respective steps are typical within the respective ranges usable. As indicated at the beginning, the main constituent of the organic matter can be considered to consist of a number of organic compounds having the general formula $C_6-H_{12}-O_6$. The gasification step converts these to the above-mentioned first gas mixture, comprising water vapour, carbon monoxide, carbon dioxide, ammonia and nitrogen, possibly with small amounts of other materials. Treatment of the first gas mixture in the first catalytic stage converts the bulk of the carbon-containing components of the mixture to dimethyl ketone and methanol and further conversion of this kind, i.e. formation of compounds having longer carbon chains or at least higher numbers of carbonations, is effected in the second catalytic treatment step. Some element of nitration of the mixture by virtue of the nitrogen-containing compounds included in it appears to occur as the vapours are subjected to the electrostatic field. This is taken further if there is a subsequent chemical nitrogen step in the overall process, though this is not usually included. The liquid fuel product can be regarded as an optionally nitrated solution of dimethyl ether and methanol, the main nitrated product presumably being methyl nitrate derived from nitration of the methanol content of the product produced after completion of the successive catalytic treatment stages.

In a preferred embodiment of the process of the invention, step (a) is carried out in the presence of one or more catalysts capable, under the conditions of temperature and pressure employed, of converting organic compounds having a given range of numbers of carbon atoms therein to organic compounds having a higher range of numbers of carbon atoms. Most preferably, step (a) comprises a first catalytic treatment of the first gas mixture in the presence of a catalyst comprising calcium oxide and alumina and a second catalytic treatment of the resultant product in the presence of a catalyst comprising calcium oxide, carbon and metallic copper, so as to form the second gas mixture, whereby the product of the first treatment has a higher range of number of carbon atoms in the molecule than the first gas mixture and the second gas mixture has a still higher range of number of carbon atoms.

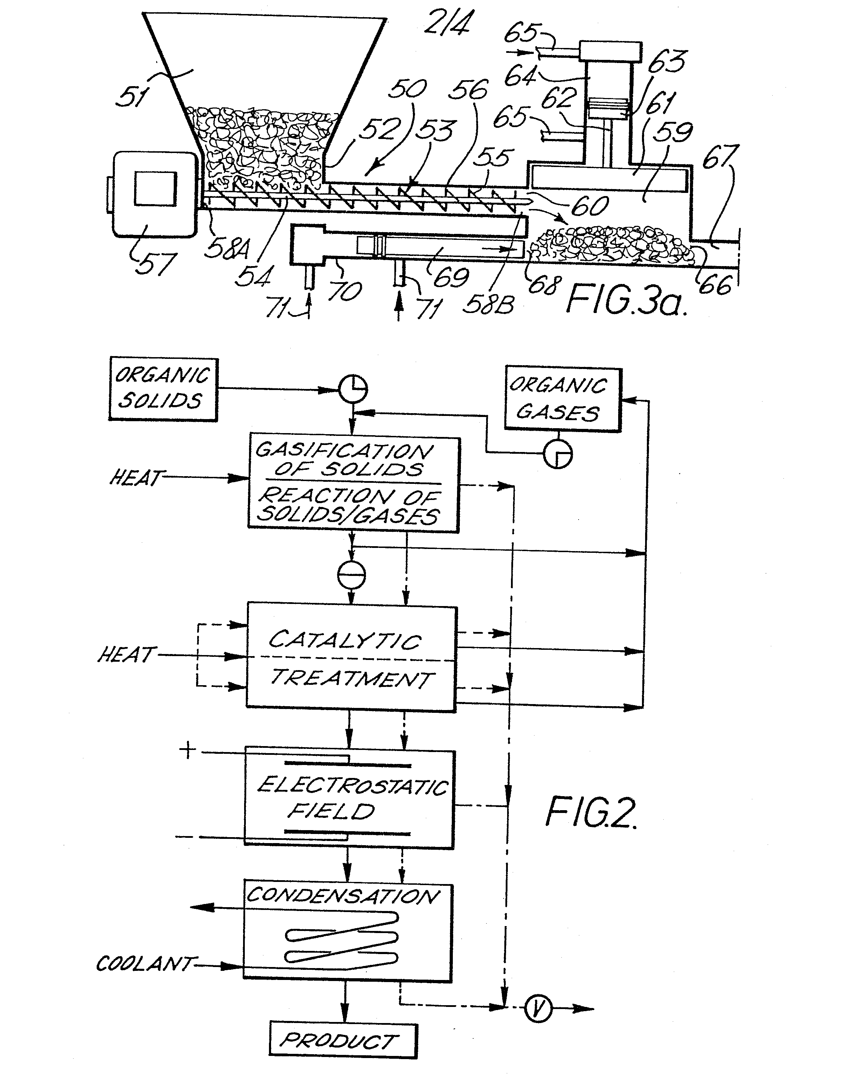

Fig. 2 illustrates in diagrammatic flow-sheet form several preferred embodiments of the process and apparatus of the invention. A sequence of reactors are involved, which respectively carry out, with heat and under pressure, either the gasification of a solid feedstock or the reaction of solid carbonaceous residues from previous gasification steps with gaseous organic feedstock, then one or more catalytic treatments, as just described, followed by exposure of the gaseous product to the electrostatic energy field and, finally, condensation or some equivalent step returning the reaction product to ambient pressure and temperature and thereby forming it into a liquid composition. The first step involves the introduction either of solid feedstock or gaseous feedstock. Valves are indicated diagrammatically, as also is the input of heat, whilst the arrowed chain-dotted lines leading to the vacuum take-off valve V in the lower right corner indicate in schematic form that the reactors can be connected to a source of vacuum for oxygen removal purposes, either in series or in parallel or in any combination thereof. The arrowed solid lines extending from the outputs from the first two reactors indicate different forms of the total energy systems included in this invention, wherein part or all of one of the gas products of the process is recycled to produce the heat energy and, by conversion, other forms of energy needed to maintain operation of the process and system.

General Reaction Sequence – Use of apparatus shown

in Fig. 1

| Step | Probable Chemical Composition | Temperature | | Pressure | | |
|---|---|---|---|---|---|---|
| | | Relative to Next Step | Range in $^{\circ}C$ | Relative to Previous Step | Psig | Range $kN/m^2$ |
| (a) Gasification | $C_6H_{12}O_6 \longrightarrow$ $CO,CO_2,H_2O,$ $NH_3,N_2$ | Not less than | 1200-1500 | Not more than | 450- 1000 | 3000- 7000 |
| (b) First Catalytic Cracking and Reformation | $N_2 + (CH_3)_2O +$ $CH_3OH + C_1-C_4$ compounds | Not less than | 300 - 750 | Not more than | 450- 900 | 3000- 6300 |
| (c) Second Catalytic Cracking and Reformation | $N_2 + (CH_3)_2O +$ $CH_3OH + NO_2 +$ $C_5-C_8$ | Not less than | 250-350 | Not more than | 450- 900 | 3000- 6300 |
| (d) Electrostatic Field Effect | As in (c) + $C_6 - C_{12}$ | Not less than | 100-200 | Not more than | 450- 900 | 3000- 6300 |
| (e) Condensation | As in (d) | – | 10 – 25 | product is liquid | | |

Another aspect of the invention resides in the apparatus, typified by that illustrated in Fig. 1 of the drawing, for carrying out the process of the invention and consisting essentially of a gasification chamber having means for injecting or otherwise introducing ground organic feedstock matter and for releasing the products of gasification of the organic matter when a predetermined pressure has been attained, a catalytic treatment chamber or, preferably, two (or even more) successive catalytic treatment chambers, containing catalysts of the kind indicated above, an electrostatic field unit for subjecting the product to the energy field, leading to a condenser which delivers a liquid product to a receptacle which either serves as a storage receptacle for the product or can introduce the product into a reactor for effecting an optional final nitration step of the process, prior to delivery of the product to the liquid storage receptacle.

Steps (a) to (c) in the reaction sequence are preferably conducted at the same elevated pressure, not less than 450 psig (3000 $kN/m^2$), achieved as a result of the gasification step (a) and maintained by setting the valves, e.g. the valves 16, 25 and 27 (Fig. 1) to open at the selected elevated pressure. The exclusion of atmospheric oxygen from the apparatus is very important and, although in theory this could be achieved by sweeping out the apparatus with an inert gas such as nitrogen before starting up the reaction sequence, it has been found that operation proceeds much more satisfactorily, as indicated above, if the apparatus is first evacuated and the atmosphere produced in each chamber in turn is that evolved in the gasification and later steps.

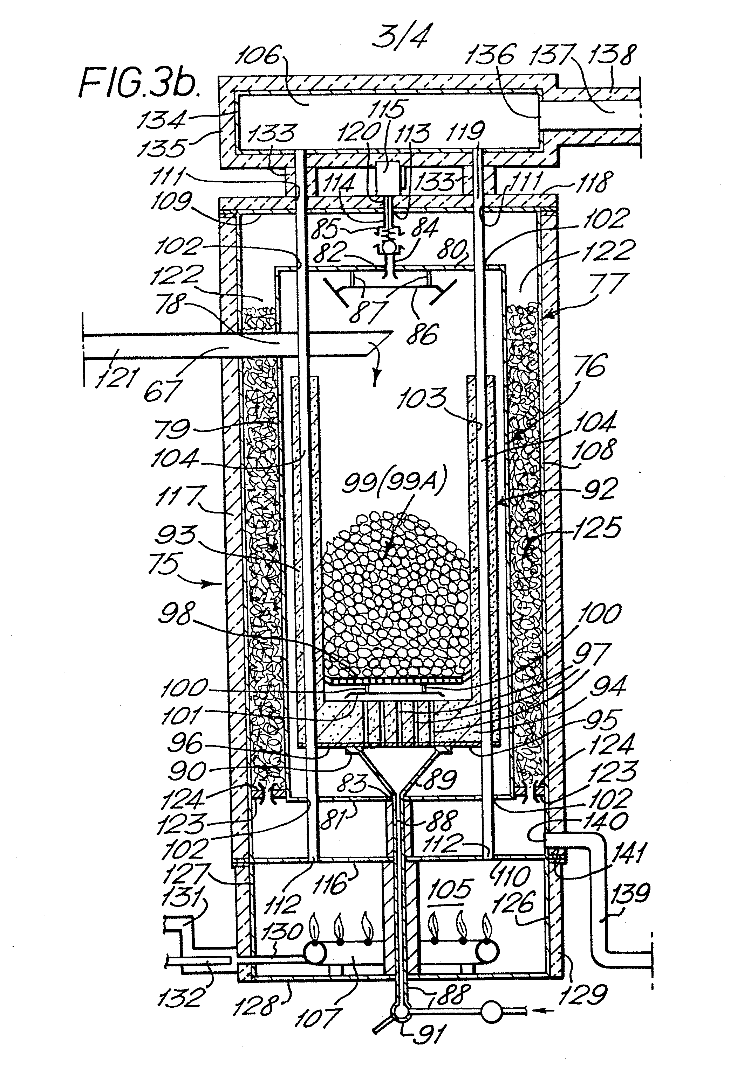

A preferred embodiment of the process of the invention, involving no nitration step, and a preferred embodiment of an apparatus for carrying it out are described below in conjunction with the three components, Figs. 3a to 3c, making up Fig. 3 of the drawings. Fig. 3a shows a unit for receiving, comminuting and introducing charges of feedstock into a thermal converter containing a first reaction zone formed within a gasification chamber; Fig. 3b shows on the same scale the thermal converter with the gasification chamber and, surrounding the latter, a second reaction zone constituting a first catalytic treatment chamber; Fig. 3c shows on the same scale a reactor tower comprising, in its lower part, a third reaction zone constituting a second catalytic treatment chamber and, in its upper part, a fourth reaction zone constituting an electrostatic energy field unit and, connected to the upper outlet from the energy field unit, a condenser unit.

Referring to Fig. 3a, the charging unit, shown generally at 50, consists of a hopper 51 for receiving any desired material, generally of an organic nature, i.e. essentially from the vegetable and/or animal kingdoms, for operating the process. The hopper 51 has its lower opening 52 arranged above the inlet end 58A of a feeder screw 53, consisting of a shaft 54 carrying a feeding and comminuting screw blade 55 and located in an elongated tubular housing 56. The feeder screw 53 operates to comminute any material received from the hopper 51 and advance it along the housing 56. The feeder screw 53 is operated by a power unit, such as an electric motor 57 attached to the inlet end 58A of the screw shaft 54 where it projects beyond the inlet end of the housing 56. The outlet or discharge end 58B of the housing 56 is attached to a feedstock charging container 59, in the form of a chamber or housing, into which the housing 56 discharges via an opening 60. Above the opening 60, the container has a vertically-movable vapour-lock piston 61 attached by a rod 62 to a hydraulic piston 63 which is vertically reciprocable inside a cylinder unit 64. The hydraulic piston/cylinder assembly 63,64 includes connections 65 to a source of hydraulic fluid (not shown) for actuating the piston 63 and therefore the vapour-lock piston 61. The feedstock charging container 59 is the cylinder of the piston/cylinder assembly 61,59, the purpose of which is to receive ground feedstock supplied by the feeder screw 53 via the housing 56 and compress it within the lower part of the container 59. This lower part communicates via an opening 66 with a feedstock injector tube 67 and also via a diametrically-opposite opening 68 with a feedstock injection piston 69. The latter is housed in a cylinder 70 under the action of the pressure of hydraulic fluid, derived from the same or a different source of pressurized hydraulic fluid (not shown) via connections 71. The preparation and introduction of a feedstock charge by operation of the unit 50 shown in Fig. 3a is carried out by putting organic material into the hopper 51 with the motor 57 driving the feeder screw 53, so as to convey the material in ground form into the chamber of the container 59, where actuation of the piston/cylinder unit 63,64 causes the piston 61 to compress the charge in the lower part of the container 59 and also seal the latter from communication with the exterior, e.g. via the feeder screw housing 56 and the hopper 51. Actuation of the piston/cylinder unit 69,70 causes the injection piston 69 to advance across the container 59 and force the compressed comminuted charge into the injector tube 67 and so into the first reaction zone, as described below. The charging unit 50 is operated until a sufficient number of charges have been introduced into the first reaction zone to form the first feedstock batch required to carry out the process of the invention.

Referring to Fig. 3b, the thermal converter unit, shown generally at 75, contains two of the reaction zones used in carrying out the process. One receives the charge from the unit 50 and subjects it to the gasification step, while the other receives the first gas mixture so produced and subjects it to the first catalytic treatment step. The gasification is carried out in a reaction zone within a decomposition chamber 76 and the catalytic treatment is carried out in a reaction zone within a first catalytic treatment chamber 77. The decomposition chamber 76 and the catalytic treatment chamber 77 are both constituted by hollow steel cylinders, that forming the chamber 76 preferably being heavy-gauge stainless steel, and the chamber 76 is located concentrically, within the chamber 77, and also so that there is a substantial annular space between the two chambers, in which the catalyst is located as described in more detail below, and also spaces at each end. The decomposition chamber 76 contains an aperture 78 in its wall 79 near its upper end, through which the injector tube 67 (Fig.3a) passes, so as to discharge the organic material into the first reaction zone within the chamber 76. Stainless steel plates 80 and 81 respectively form the upper and lower ends of the chamber 76 and a central aperture 82 is made in the upper plate 80 and another central aperture 83 is made in the lower plate 81. An outlet tube 84 passes through the aperture 82 and terminates in a solenoid-actuated or other remotely-controllable pressure vent valve 85. Inside the upper part of the chamber 76, a steel disc 86 is mounted on spacer bars 87 so as to shield the end of the outlet tube 84. An inlet tube 88 passes through the aperture 83 and supports a conical feed gas shield funnel 89 having an annular supporting flange 90 around its upper rim. The inlet tube 88 extends downwardly out of the lower end of the unit 75 and connects via a gas feed control valve 91 with a gas feed source (not shown). An open-topped cylindrical graphite decomposition chamber or pot 92 is mounted within the chamber 76 so as to be concentric with it. The pot 92 has an integral cylindrical wall 93 and a thick base 94 and rests with the lower surface 95 of its base 94 on the flange 90. A circular steel disc 96 can be welded to the flange 90 and have a diameter equal to that of the pot 92, so that the latter is supported over the whole of the area of its base 94. Within the circular area of the base 94 inside the funnel flange 90, the pot base 94 and, if provided, the disc 96 have a number of through-going gas vents 97, via which the gas inlet tube 88 communicates with the inside of the pot 92, by way of the funnel 89 and the vent holes 97 in the pot base 94. Within the pot 92, a perforated plate 98 for supporting a mass 99 of particulate catalyst is provided, preferably by being force-fitted or otherwise supported, and on its underside dependent spacer bars 100 support a circular feed gas deflector plate 101, which is located above the area of the pot base 94 containing the vent holes 97. Thus, gas from the source (not shown) can be passed via the tube 88 under the control of the valve 91 to the funnel 90 and then via the vent holes 97 to the inside of the graphite pot 92, where it is directed outwardly by the deflector plate 101 and can then pass via the perforated plate 98 throughout the mass of particulate or otherwise gas-permeable material supported on the plate 98 after the gasification of a charge of organic matter, as described below.

The chamber 76, the graphite pot 92 in it and the charge of material, 99A, from which the mass 99 of residue is formed, are raised to and maintained at the requisite temperature by indirect heating. Any form of heating can be used and Fig. 3b shows the use of indirect gas-fired heating. The end plates 80 and 81 of the chamber 76 contain correspondingly arranged series of apertures 102 and these match up with each other and also with similar series of apertures, described below, made in the end plates, also described below, of the catalytic treatment chamber 77. The apertures 102 are also aligned with throughgoing holes 103 in the thickness of the wall 93 of the pot 92. Steel flame tubes 104 pass through the treatment chamber 77 from a burner chamber 105 at the lower end of the converter unit 75, as described below, to a heat expansion chamber 106 at its upper end, also as described below. These flame tubes 104 thus pass also completely through the chamber 76 by way of the ring of apertures 102 in the lower end plate 81, the holes 103 in the pot 92 and the ring of apertures 102 in the upper end plate 80. Gasification is carried out with the gas feed control valve 91 closed, with the vent valve 85 in the outlet tube 84 from the chamber 76 also closed and with the charging unit 50 closed by the piston 69 sealing the injection tube 67 and/or the piston 61 in the vapour-lock position and sealing the chamber 59 from the tubular housing 56 and thus the hopper 51. The heat necessary for effecting gasification is derived from a gas burner unit 107 located in the burner chamber 105, described in more detail below, and passes through the ring of flame tubes 104. Since the decomposition chamber 76 and the other reaction zones of the apparatus are effectively free of oxygen during operation of the process, the high temperature in the chamber 76 and the solid construction of the pot 92 cause little if any decomposition of the graphite structure. This also applies to the preferred alternative stage in which an organic gas is fed into the hot chamber 76 after conclusion of the gasification step, in order to remove the finely-divided solid residues represented by the mass 99 and produce additional quantities of gas mixture for processing in the later steps into further liquid composition. Although the bulk of the mass 99 is carbon and is thus essentially the same elemental material as the pot 92, it is the mass 99, which is predominantly removed by reacting with the gas and forming more liquid composition because of its finely-divided character and other physical attributes.

The catalytic treatment chamber 77 consists of a cylindrical steel tube 108 closed at each end by circular plates 109 (upper) and 110 (lower). Each of the plates 109,110 contains a circular array of apertures 111 (upper plate 109) and 112 (lower plate 110) for receiving the flame tubes 104, which pass through the apertures 111 in the upper plate 109 and project into the heat expansion chamber 106 and, at their lower ends, the flame tubes 104 are welded to the plate 110 at the apertures 112. Another aperture 113 in the centre of the upper plate 109 accommodates the actuating rod 114 of the vent valve 85, under the control of an electrical actuator 115, while an aperture 116 is provided in the centre of the lower plate 110 to allow passage of the gas inlet tube 88, from outside the convertor unit 75 to the interior of the chamber 76. The tube 108 is wholly encased in a heavy thermal insulation jacket 117 and a further circular insulation slab 118, having apertures 119 for the flame tubes 104 and an aperture 120 for the rod 114, is applied over the upper plate 109. Near the upper end of the chamber 77, an aperture 121 is aligned with the aperture 78 in the chamber 76 to allow the injector tube 67 of the charging unit 50 to pass through the insulation jacket 117 and the tube 108 and across the annular space 122 between the chambers 76 and 77 and into the decomposition chamber 76. At the bottom of the chamber 76, it is supported inside the chamber 77 by mounting struts 123, which also support permeable means, such as an annular mesh grid 124 upon which the catalyst mass 125 is carried. The lower plate 110 is not provided with an insulation slab like the slab 118, because a separate cylindrical housing 126, comprising a drum-like wall 127 and a circular base plate 128 is mounted under the lower plate 110 and forms the burner chamber 105. A tubular insulation jacket 129 surrounds the wall 127 of the burner chamber 105 and thermal insulation units 130 lag the inlet tube 88 where it is located between the base plate 128 and the lower plate 110, in the housing 126, and between the lower plate 110 and the lower plate 81. Within the burner chamber 1-5, the annular gas burner unit is located, being supported for instance upon the base plate 128, and is connected to a source of gas (not shown) by a gas feed tube 130 connected to a combustion air inlet branch 131 containing a gas inlet nozzle 132. At the top of the catalyst chamber 122, the flame tubes 104 are surrounded by insulation blocks 133 where they pass to the heat expansion chamber 106 and this is a steel drum-like housing 134 of the same general circular cross-section as the chamber 77 and it is surrounded on all sides by a heavy insulation jacket 135 except at an aperture 136 in the side of the housing 134 which communicates with an outlet flue 137 also surrounded with thermal insulation 138. At the bottom end of the chamber 77, below the grid 124, a gas transfer tube 139 passes from an aperture 140 in the tubular wall 108 of the chamber and through an aperture 141 in the insulation jacket 117, to the bottom end of the reactor tower shown in Fig. 3C, as described below. The outlet flue 137 from the heat expansion chamber 106 is connected to the top of the reactor tower of Fig. 3C, as will appear from the more detailed description below. Means for monitoring the pressure and temperature in the various reactor zones of the apparatus are provided together with electrical circuitry for operation of the vent valve actuator, 115 and operation of the hydraulic piston/ cylinder assemblies of Fig. 3a, but these components have not been shown in order to ensure that Figs. 3a, 3b and 3C show the parts described in detail with sufficient clarity.

Referring to Fig. 3c, the reactor tower, shown generally at 150, contains two of the reaction zones used in carrying out this embodiment of the process of the invention. One is the second catalytic treatment chamber 151, forming the lower part of the tower 150, and the other is the electrostatic energy field unit 152, forming the upper part of the tower 150. Fig. 3c also shows the final unit in the apparatus of this embodiment, namely the condenser 153.

The reactor tower 150 consists of a superposed pair of similar cylindrical housings made of sheet steel, the lower housing 154 comprising a tubular structure with a lower end plate 155 and an apertured upper end plate 156 and the cylindrical housing 154 is surrounded by a tubular thermal insulating jacket 157 and a circular insulating slab 158 covers the underside of the end plate 155. An aperture 159 in the housing 154 adjacent the end plate 155 and a mating aperture 159A in the jacket 157 allow the gas transfer tube 139 (Fig. 3b) to be connected from the bottom of the first catalytic treatment chamber 77 to the bottom of the second catalytic treatment chamber 151. Above the aperture 159, a perforated support plate 160 is mounted across the catalyst chamber 151 and a mass of catalyst particles, shown at 161, is supported on the plate 160 and substantially fills the reaction zone formed by the annular space within the housing 134 surrounding the outlet flue 137 from the heat expansion chamber 106 at the upper end of the thermal converter unit 75 (Fig. 3b). This outlet flue 137 is in the form of an elongated pipe of substantial internal cross-section, which extends horizontally from the chamber 106 through a short distance and then passes vertically downwards through the reactor tower 150 and terminates in a lower outlet 162, which forms the vent from the burner chamber 105 via the flame tubes 104 and the chamber 106. This arrangement utilizes the heat in the exhaust gases to raise the temperature within the catalyst chamber 151 and the electrostatic field unit 152, as explained in detail below. The outlet 162 can be vented to the atmosphere.

Above the chamber 151 containing the catalyst 161 for effecting the second catalytic treatment of the product of the gasification step effected in the chamber 76, the electrostatic field effect unit 152 is located. It consists of the housing 152, having a perforated lower end plate 163 which allows the gas product leaving the chamber 151 to pass into the housing 152 and a similar apertured upper end plate 164. The cylindrical housing 152 is thermally insulated by means of an exterior jacket 165 and, further, an electrically insulating layer 166 covers the exterior of the length of the flue 137 which passes through the housing 152 from the upper end plate 164 to the lower end plate 163. An upper outlet chamber 167, consisting of a drum-shaped housing 168 having a circular upper end plate 169 and an apertured lower end plate 170 matching the end plate 164 of the housing of the firld effect unit 152, is located at the top of the reactor tower 150. Thermal insulation in the form of a jacket 171 covers the outer surfaces of the housing 168 and abuts the insulation 138 surrounding the outlet flue 137 where it forms an elbow between the horizontal exit portion from the expansion chamber 106 and the top of its vertical portion passing down through the unit 152 and then the catalytic treatment chamber 151. The housing 168 includes a lateral aperture 172 forming the entry to an outlet duct 173 which passes through another aperture 174 in the jacket 171 and serves to convey the gaseous mixture forming the product leaving the unit 152, so as to convey such product to the condenser 153.

In order to generate the electrostatic field required to effect the gas mixture leaving the second catalytic treatment chamber 151 via the apertures in the end plates 156,163 as it passes through the unit 152 and is then discharged to the outlet chamber 167 via the apertures in the end plates 164,170 and proceeds to the condenser 153 via the outlet duct 173, an open-ended tubular positive electrode plate 175 is mounted upon the exterior of the outlet flue 137 within the unit 152, being located upon the insulating layer 166 and a corresponding open-ended tubular negative electrode plate 176 surrounding and concentrically-spaced from the positive electrode plate 175 is mounted upon the interior surface of the housing 152A of the field unit 152. The negative electrode plate 176 can extend throughout the height of the unit 152 from the end plate 163 at the bottom to that, 164, at the top, but the positive electrode plate 175 is advantageously disposed so that it is spaced at the bottom and top from the respective end plate 163, 164, by means of lower and upper spark insulators 177,178. The negative potential input to the unit 152 can be provided in any desired way, such as by means of a negative connecting lead 179 attached to the plate 176 inside the unit 152 and to a mains connecting terminal 180 located in a suitably insulated manner on the exterior of the jacket 165. The positive connection to the electrode plate 175 extends from the upper end of the latter and passes out of the unit 152 via an insulating grommet 181 located in the end plate 170 and another such grommet 182 located in an aperture in the upper end plate 169 of the housing 168, the connection itself being indicated at 183. In practice, it connects the electrode plate 175 with a positive terminal 184 located in an insulated manner on the exterior of the insulating jacket 171. The positive and negative terminals 184, 180 are connected to a source of direct current voltage (not shown) capable of providing current at a voltage in the range, preferably,

0125067

- 28 -

from 25,000 to 40,000 volts and most preferably of 30,000 volts. The annular space 185 located between the concentric and mutually-facing electrodes 175,176 constitutes the means by which the gaseous product leaving the chamber 151 reaches the outlet chamber 167 and passes to the condenser 153 and, as the product is subjected to the electrostatic field produced by the potential difference across this space 185, a further stage of chemical modification takes place.

The condenser 153 consists of a long tubular housing 186 having upper and lower end plates 187,188 and upper and lower connections 189,190 for discharging and receiving coolant, e.g. water at room temperature, from a coolant source (not shown). The upper discharge connection 189 preferably includes a coolant control valve 191. The coolant passes through coolant passageways 192 in the condenser 153 and the gas product arriving at the top of the condenser 153 via the duct 173 condenses on the surfaces of these passageways 192 and undergoes liquification and the product is reduced to normal temperature and pressure. At the foot of the condenser 153, a fluid take-off line 193 is provided and incorporates a pressure valve 194, together with a branch connection 195, including a control valve 196, which is coupled to a vacuum source (not shown).

In order to carry out the process according to the present invention in the apparatus described above in relation to Fig. 3 (Figs. 3a to 3c together), the preferred mode of operation is as follows:

OPERATION WITH ORGANIC SOLIDS

Feedstock in the form or organic solids of any origin, preferably having a moisture content up to 65% down to 0.01% by weight, is placed in the hopper 51 of the charging unit 50, up to the required level, and is then reduced in particle size by the comminuting action of the revolving feeder screw 53 and carried by it through the housing 56 to the feedstock charging container 59. Once the charging chamber 59 is full, the vapour-lock piston 61 compresses the feedstock into a form suitable for entry into the injector tube 67, where it is injected into the graphite pot 92 in the decomposition chamber 76 by means of the injection piston 69. This system acts as an effective air and gas-tight seal; alternative means for introducing charges of feedstock can be employed if so desired.

Once the decomposition chamber 76 has been loaded with sufficient feedstock, in the form of the charge 99A, and is sealed by the forward movement of the injection piston 69, the whole system is then evacuated to a reduced pressure of 1 to 2 mb (0.1 to 0.2 $kN/m^2$), for instance 0.13 $kN/m^2$ or approximately 1 mm of mercury, in order to remove atmospheric gases and thus molecular oxygen from the apparatus, which is then sealed. This is done by closing the communication between the chamber 76 and the exterior via the housing 67 by means of either or both of the pistons 61 and 69, opening the valve 85, closing the pressure valve 194 and coupling the vacuum source (not shown) to the branch connection 195, with the valve 196 open; after the desired evacuation of the series of reaction zones has been carried out in this way, the apparatus is sealed by closing the valve 196. The vent valve 85 is also closed, so as to close the decomposition chamber 76. Heat is then generated by means of the gas burner unit 107 and the decomposition chamber 76 and thus the graphite pot 92 and the charge 99A are raised to a temperature of $1000^{\circ}$ to $1500^{\circ}$C by means of the heat passing through the flame tubes 104.

As the temperature of the pot 92 is raised, the feedstock 99A is decomposed, creating an autogenous and increasing gas pressure within the decomposition chamber 76, due to the expansion of the feedstock matter, as it is changed from the solid state to liquid materials and gases. This continues until the whole mass 99A of the feedstock charge is converted to gaseous products, i.e. the first gas mixture described above, and the predominantly carbon residue 99.

At a predetermined pressure, for instance in the range from 60-100 psig, i.e. 420-700 $kN/m^2$, the gaseous products react to form carbon monoxide, hydrogen, traces of ammonia and traces of carbon dioxide, together with traces of superheated water vapour and nitrogen. As the temperature and pressure increase, the gaseous products react further and produce carbon monoxide, hydrogen, methane and traces of other hydrocarbon gases and also traces of free nitrogen.

At a pressure of 100 psi (675 $kN/m^2$), the electrically-operated vapour vent valve 85 at the top of the decomposition chamber 76 is opened and allows passage of the gaseous products into the first catalyst chamber 77 which is maintained at a temperature of $600^{\circ}$-$800^{\circ}$C by the heat generated in the decomposition chamber 76 and transmitted through the walls 108, 109, 110 and also directly from the burner chamber 105 via the lower plate 110. The gaseous product is forced through the bed 99A of catalytic compound granules where the gaseous product is converted to a mixture of methane and hydrocarbon vapours with a substantial removal of oxygen. From here, the mixture of gas and hydrocarbon vapours is transferred through the vapour transfer tube 139 to the base of the reactor tower 150.

Once in the reaction tower 150, the gas and vapour mixture is forced up through the further bed 161 of catalyst compound which is maintained at a temperature of $200^{\circ}$-$400^{\circ}$C by the heat supplied from the exhaust tube or outlet flue 137. On ascending through the bed 161 of catalyst compound, further reactions are promoted, converting substantially the whole of the gas and vapour mixture to hydrocarbon vapours in the $C_5$ to $C_{10}$ range. The gas mixture is then passed through the electrostatic force field generated by the potential difference of 25,000 to 40,000 volts DC across the electrodes 175,176. In this unit, the energy levels of the constituents of the gas mixture appear to be momentarily raised above the ground state, causing partial decomposition of the molecules in the lower carbon number range. As the energy levels return to the ground state, any unsaturated hydrocarbon chains are saturated and the smaller molecular fragments add on to the larger chains, forming a mixture of fully saturated hydrocarbon chains in the range $C_8$-$C_{12}$.

On passing through the electrostatic force field in the unit 152, the product is then transferred through the outlet duct 173 to the condenser unit 153, where the product is condensed to a liquid hydrocarbon containing various fractions, typically in the range from $C_8$-$C_{12}$. The product proves to be a comparable alternative fuel to that of fossil fuel originated fractions. When the gasification step has been completed and the product has undergone the catalytic treatments, exposure to the electrostatic energy field and condensation, the plant may be shut down for removal of the largely carbon-containing residue in the housing of the decomposition chamber 70. The process may then be repeated. It is also possible to put a further feedstock charge into the chamber 76 and repeat the process until the pot 92 is filled with the carbon-based residue and so has to be emptied or otherwise treated.

OPERATION WITH GAS FEEDSTOCK

The plant may also be used to convert natural gas to hydrocarbon liquid by the use of an alternating feedstock input programme in the following way.

As the liquid product is drawn off from the condenser 153 and the pressure is again reduced in the decomposition chamber 76, the charging unit 50 is not operated and is kept closed so that there is no communication through the housing 56 from the chamber 76 to the atmosphere. A charge of natural gas is then introduced into the still-hot chamber 76 through the gas feed source input system, by opening the gas control valve 91. The gas then passes up through the inlet tube 88 and via the funnel 89 to the feed gas vents 97 in the base 94 of the graphite pot 92, it is distributed by the feed gas vent shield 101 and passes through the perforated grate or plate 98 and so percolates through the bed or mass 99 of residue carbon left by the solids feedstock, which is still at a temperature of $1000^{\circ} - 1500^{\circ}C$. This is continued, with the vent valve 85 closed, until a pressure of 60 - 100 psi (400 - 675 $kN/m^2$) is achieved in the chamber 76. The gas reacts with the residual carbon and dissociates the methane present. The gas supply is turned off by closing the valve 91 and the vapour vent valve 85 is then operated at approximately 100 psi (675 $kN/m^2$), so allowing the products of reaction of the residue with the gas at elevated temperature and pressure in the effective absence of molecular oxygen to form and then pass into the catalyst chamber 77, where reactions occur promoting the formation of long-chain hydrocarbon molecules and the removal of trace elements such as oxygen. On passing from the catalyst 77, the catalyst chamber 151 and the electrostatic force field in the unit 152, essentially the same reactions are promoted as occur when the gasification step is carried out. Condensation of the product again produces a compound liquid as before. In this alternative phase of operation however, the residual carbon which is left in the decomposition chamber 76 during the operation with solid feedstock is substantially entirely removed by reaction with the gas feedstock, leaving only a minor amoung of residue in the pot 92, which is essentially mineral in nature and consists of the oxides of any metals present in the original solid feedstock together with any other traces of compounds which are resistant to either gasification or the alternative carbon/natural gas reaction phase. Operation with alternative solid and gaseous feedstock ensures substantially continuous operation of the apparatus to produce a combustible liquid product. The plant will gradually accumulate some amount of permanent residue, which can be withdrawn at intervals, for instance by opening up the chamber 77 at the top and then the chamber 76 and extracting the residue from the plate 98, for instance by suction; an alternative way to remove any residue is to introduce a suction line into the pot 92 via the injector tube 67,

In any operation of apparatus according to the invention or in carrying out any form of the process of the invention, to make the product of the invention, a number of important points should be observed:

1. Once the first charge of solid feedstock has entered the decomposition chamber 76 and before heat is applied, the whole system is evacuated e.g. via the line 33 (Fig.1) or via the vacuum take-off 195 (Fig. 3c), so that all atmospheric gases are removed and the plant is reduced to a pressure of 1 mm Hg (1-2 mb). If this were not done, the reactions described would not occur and there would be a danger of coating the catalyst 125 in the chamber 77 with tar, resulting in excess pressure being maintained in parts of the system. Therefore, safety devices (not shown) are preferably installed to prevent operation of the initial start-up cycle unless the pressure is at a desired starting pressure, such as 1 mm Hg (1-2 mb).

2. The vapour vent valve 85 located in the top of the decomposition chamber 76 is advantageously coupled to a pressure sensing circuit and this is pre-set to operate within a given pressure range, i.e. to open at 100 psi and to close at 15 psi (to open at 700 and close at 100 $kN/m^2$. This prevents the formation of tar, which would otherwise be carried over and deposited on the catalyst 125. On reaching a temperature of 800$^o$C and a pressure of 20 psi, (about 140 $kN/m^2$), any formed would decompose to hydrocarbon gases of much smaller molecular size.

3. Advantageously, the feedstock injection is operated on a cyclic basis, so as to allow one charge at a time of predetermined mass to enter the chamber 76; with such an arrangement, a further charge can only enter when the pressure in the chamber 76 has fallen, say, to 15 psi (100 $kN/m^2$) or below. If the plant is operated as a dual process plant with natural gas feed, then the preferred cycle of operation is to use one charge of solids and then one of gas, one of solids, one of gas and so on. An input of solids and gas together cannot be employed, as gasification would not then take place as desired; advantageously, this is prevented by an electronic interlock circuit, which ensures that the charging unit 50 and the gas control valve 91 cannot be opened at the same time.

On an initial cold start, the whole system must be evacuated as described to prevent entry of atmospheric gases. However, once the process is in operation, either with all solids or with solids and gas alternately, the system will then generate continuously without the need for further evacuation, as the only gases present throughout the system will be those which are purposely produced on injection and gasification.

Reactivation of the catalysts can be achieved, when required, by passing hydrogen through the system by means of the gas feed line 88. This produces water vapour and carbon dioxide which are then vented through the fluid take-off point 193 and the valve 194. Reactivation is required at approximate intervals of 20-30 hours, dependent upon the feedstock; however reactivation can be automatically indicated when it is required, by appropriate sensing circuits contained in the main control system.

The invention has great versatility and can be embodied in forms of apparatus of widely differing kinds. For example, in rural areas where farming methods yield large amounts of waste organic matter and where liquid fuel normally has to be brought in from distribution centres by tanker, it is possible to construct the apparatus according to the invention as a mobile organic matter converting plant. The various components of the apparatus, such as shown in the embodiment of Fig. 1 or that of Figs. 3a to 3c, can be mounted upon one or more truck bodies and brought to rural sites at intervals. The provision of one or more liquid fuel storage tanks and possibly a storage and grinding centre for organic waste matter enables the truck-mounted plant to be operated, either with connection to a local electricity supply or, if more convenient, the mobile plant can itself include a generator. As soon as the plant is ready for operation, multiple-batch gasification is commenced. If a piped or bottled gas supply is also available, the plant can operate with alternate solid and gas feed, until the accumulated waste organic matter has all been consumed and converted into liquid fuel which is left in the local storage tanks, which can feed the fuel as required to vehicles or fixed units, including heater units for greenhouses and other buildings. Thus, regular visits to the area by the mobile plant ensure that adequate supplies of fuel are continuously available and, moreover, that the problem of the disposal of organic waste matter produced in other farming operations is also solved.

Another form of operation would involve a fixed centralized conversion plant which can serve as a collecting and processing centre for waste matter for a large area.  Those using the system can, in effect, exchange their unwanted organic waste materials for supplies of liquid fuel made from such material on a continuous multiple-batch alternating-feedstock principle, such as is described in conjunction with Figs. 2 and 3a to 3c.

CLAIMS:

1. A process of production of a liquid composition, useful as a fuel, which comprises:

(a) subjecting a first organic gas mixture to at least one stage of catalytic treatment at elevated temperature and elevated pressure so as to cause interaction of the components of the first gas mixture and formation of a second organic gas mixture, and

(b) exposing the second gas mixture to an electrostatic energy field of the silent discharge type, wherein steps (a) and (b) are each conducted in the substantial absence of molecular oxygen, step (b) is conducted at an elevated pressure not greater than the maximum pressure attained in step (a) and step (a) is conducted at an elevated temperature not less than that used in step (b).

2. A process according to claim 1, wherein the first gas mixture is produced by subjecting finely-divided solid organic matter to decomposition at elevated temperature under autogenous pressure, so as to effect substantially complete gasification of the organic matter, and such gasification step is carried out in the substantial absence of molecular oxygen and at an elevated temperature not less than that used in step (a).

3. A process according to claim 1 or 2, wherein step (a) is carried out in the presence of one or more catalysts capable, under the conditions of temperature and pressure employed, of converting organic compounds having a given range of numbers of carbon atoms therein to organic compounds having a higher range of numbers of carbon atoms.

4. A process according to any of claims 1 to 3, wherein step (a) comprises a first catalytic treatment of the first gas mixture in the presence of a catalyst comprising calcium oxide and alumina and a second catalytic treatment of the resultant product in the presence of a catalyst comprising calcium oxide, carbon and metallic copper, so as to form the second gas mixture, whereby the product of the first treatment has a higher range of number of carbon atoms in the molecule than the first gas mixture and the second gas mixture has a still higher range of number of carbon atoms.

5. A process according to claim 4, wherein the first catalyst comprises, by weight, 50% - 70% of CaO and 30% - 50% of $Al_2O_3$, together with from 1% to 5% of graphite, and the second catalyst comprises, by weight, 30% - 50% of CaO, 30% - 50% of copper and 15% - 30% of graphite.

6. A process according to claim 5, wherein the first catalyst contains, by weight, 55% of CaO, 40% of $Al_2O_3$ and 5% of graphite and the second catalyst contains, by weight, 40% of CaO, 40% of copper and 20% of graphite.

7. A process according to any preceding claim, wherein each catalyst is provided in the form of a gas-permeable particulate mass.

8. A process according to claim 2 or any of claims 3 to 7 as dependent thereon, wherein operation is effected by a multiple batch procedure, in which a first charge of solid organic matter is subjected to fine sub-division, if necessary, and is then subjected to treatment in a reaction zone until the gasification step has been completed and the first gas mixture so formed is then transferred to a second reaction zone in order to undergo step (a).

9. A process according to claim 8, wherein after the first charge has been gasified, a second charge is then introduced and gasified, with or without removal of the residue left by gasification of the first charge.

10. A process according to claim 8 or 9, wherein, after the gasification step has been completed and while the reaction zone remains at the elevated temperature or is maintained at another elevated temperature and also while the reaction zone and the residue in it are subject to an elevated pressure derived from the autogenous pressure of the gasification step, a combustible organic gas feed is supplied to the reaction zone and reaction of it with the essentially carbonaceous residue yields a quantity of another gas mixture, which then undergoes steps (a) and (b), resulting in the production of additional liquid composition and substantial removal of the residue from the reaction zone.

11. A process according to claim 10, wherein solid feedstock charges are employed alternately with gaseous feedstock charges, any non-gasifiable residue which forms being removed.

12. A process according to any of claims 8 to 11, wherein the gasification step is carried out by subjecting the or each charge to a temperature in the range from $1000^{\circ}$ to $1500^{\circ}C$ and the autogeneous pressure is raised to a value in the range from 3500 to 7000 $kN/m^2$ prior to releasing the first gas mixture to undergo catalytic treatment.

13. A process according to claim 12, wherein the temperature is in the range from $1000^{\circ}$ to $1200^{\circ}C$ and the autogenous pressure is 5000 $kN/m^2$.

14. A process according to any preceding claim, in which the or each catalytic treatment step is carried out at a temperature in the range from $300^{\circ}$ to $750^{\circ}C$ and at a pressure in the range from 3000 to 6000 $kN/m^2$.

15. A process according to any preceding claim, wherein step (b) is carried out by subjecting the second gas mixture to an electrostatic energy field generated at 25,000 to 40,000 volts D.C., prior to cooling and condensing the resultant product to form the desired liquid composition.

16. An apparatus for producing a liquid composition useful as a fuel from organic matter, which comprises:

(a) a first reaction zone capable of being evacuated and heated, having a sealable inlet for the introduction of a charge of organic matter, heating of which causes gasification and the formation of a first gas mixture under the autogenous pressure of such gasification step, and a sealable outlet for effecting discharge of the first gas mixture from the first reaction zone,

(b) a second reaction zone capable of being heated and arranged to contain at least one catalyst substance for effecting reaction of the components of the first gas mixture to form a second gas mixture, the second reaction zone having an inlet for introduction of the first gas mixture connected to the sealable outlet from the first reaction zone and an outlet for effecting discharge of the second gas mixture from the second reaction zone,

(c) a third reaction zone including means for generating an electrostatic energy field of the silent discharge type and having an inlet for introduction of the second gas mixture connected to the outlet from the second reaction zone and an outlet for effecting discharge of a third gas mixture produced by the action of the energy field upon the second gas mixture as it passes through the third reaction zone,

(d) a condenser connected to the outlet from the third reaction zone and serving to effect reduction of the temperature of the third gas mixture to produce the desired liquid composition, and

(e) means for connecting the first, second and third reaction zones and the condenser to a source of vacuum.

17. An apparatus according to claim 16, wherein the second reaction zone comprises a first catalytic treatment zone for receiving the first gas mixture and subjecting it to a first catalytic thermal conversion to produce a modified gas mixture and a second catalytic treatment zone for receiving the modified gas mixture and subjecting it to a second catalytic thermal conversion to produce from it the second gas mixture prior to discharging it via the outlet from the second reaction zone to the third reaction zone.

18. An apparatus according to claim 16 or 17, wherein the first reaction zone is provided with a closable inlet for introducing into the first reaction zone one or more charges of organic matter to be gasified, an outlet including a pressure-release valve operable at a predetermined autogenous pressure to discharge the first gas mixture from the first reaction zone and means for heating the contents of the first reaction zone in order to subject the one or more charges of organic matter to gasification.

19. An apparatus according to claim 17 or 18, wherein the first reaction zone is located inside the first catalytic treatment zone, so that the latter is heated by the heat discharged from the first reaction zone and also by the heat of the first gas mixture discharged therefrom.

20. An apparatus according to any of claims 16 to 19, wherein the third reaction zone and the second reaction zone comprising the second catalytic treatment zone form a reactor tower which is traversed by the second gas mixture leaving the first catalytic treatment zone.

21. An apparatus according to claim 20, wherein a source of burnable gas forms the means for heating the first reaction zone and the heat is directed through a plurality of tubes which pass through the first reaction zone from a lower burner chamber to an upper heat expansion chamber and an outlet flue from the latter traverses and thus indirectly heats the third reaction zone in which the electrostatic field operates and then the second catalytic treatment chamber, an outlet for discharging the fourth gas mixture connecting the third reaction zone to the condenser.

22. An apparatus according to any of claims 16 to 21, in which a charging unit for the introduction of organic matter is connected to the sealable inlet to the first reaction zone and such unit consists of a chamber for receiving organic matter in comminuted form, a reciprocable piston for compressing such matter and sealing the chamber from communication with the atmosphere and an injection piston operable to direct compressed comminuted matter from the chamber to the inlet to the first reaction zone.

23. An apparatus according to any of claims 16 to 22, in which a connection to a source of vacuum is provided, for connecting the reaction zones to such vacuum source prior to the introduction of organic matter, so as to allow gasification of the organic matter to occur in the substantial exclusion of molecular oxygen.

24. An apparatus according to any of claims 16 to 23, wherein the first reaction zone comprises a steel chamber traversed by burner flame tubes for conveying heat to the chamber and an open-topped pot of graphite arranged to receive the charges of organic matter for gasification, whereby the residue from the gasification step is contained in the graphite pot in the form of a substantially carbonaceous finely-divided mass.

25. An apparatus according to claim 24, wherein a plurality of vent holes are provided in the base of the graphite pot for directing an organic gas feedstock into contact with the residue from the gasification step and a gas feed conduit for such organic gas feedstock connects with the plurality of vent holes whereby the residue from a gasification step is reacted with the gas to form additional quantities of a first gas mixture, prior to the resumption of operation with the organic matter.

FIG.1.

FIG.3a.

FIG.2.

FIG.3b.

0125067

4/4

FIG.3c.

**European Patent Office**

**PARTIAL EUROPEAN SEARCH REPORT**
which under Rule 45 of the European Patent Convention
shall be considered, for the purposes of subsequent
proceedings, as the European search report

0125067

Application number

EP 84 30 2795

## DOCUMENTS CONSIDERED TO BE RELEVANT

| Category | Citation of document with indication where appropriate, of relevant passages | Relevant to claim | CLASSIFICATION OF THE APPLICATION (Int Cl 3) |
|---|---|---|---|
| X | <u>FR - A - 540 543</u> (G. PATART)  <br><br> * The whole document * <br><br> ---- | 1-3,7 | C 10 L   1/00// <br> C 07 C   9/14 <br> C 07 C  31/04 <br> C 07 C  43/04 <br> C 07 C   1/02 <br> C 07 C   2/80 <br> C 07 C  29/15 <br> C 07 C  41/01 |

TECHNICAL FIELDS
SEARCHED (Int. Cl. 3)

C 07 C  29/00
C 07 C  41/00
C 10 L   1/00

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-25

Claims not searched:

Reason for the limitation of the search:

The expression "useful as a fuel" is inter-preted as non-limiting and therefore a com-plete search is not possible. A limited search based on the clearly identified products and processes mentioned in the description and on liquid fuels of unspecified chemical composition has been carried out.

| Place of search <br> The Hague | Date of completion of the search <br> 26-07-1984 | Examiner <br> WRIGHT |
|---|---|---|